# EUROPEAN PATENT SPECIFICATION

(11) **EP 2 886 089 B1**
(45) Date of publication and mention of the grant of the patent: **06.10.2021**
(21) Application number: 14197860.1
(22) Date of filing: 15.12.2014
(51) Int. Cl.: B29C 65/08, A61F 13/15, B29C 65/00, B29L 31/48

(54) **A method and apparatus for the transverse welding of overlapping elastic structures**
Verfahren and Vorrichtung zur Durchführung eines Querschweissvorganges bei überlagerten elastischen Strukturen
Procédé et appareil pour le soudage transversal des structures élastiques superposées

(30) Priority: 17.12.2013 IT TO20131031
(43) Date of publication of application: 24.06.2015
(60) Divisional application: 19183812.7
(73) Proprietor: Fameccanica.Data S.p.A., 66020 San Giovanni Teatino (CH) (IT)
(72) Inventor: Sablone, Gabriele, I-65125 Pescara (IT)
(74) Representative: Marchitelli, Mauro

(56) References cited:
- EP-A2- 1 393 701
- US-B1- 6 450 321

## Description

### Technical field

The present description refers to the transverse welding of a composite tape formed from two or more layers of overlapping elastic material, which can be used for producing sanitary articles such as training-pant products of the pre-fastened type.

### Technological background

A common technique for the production of sanitary products wearable in the manner of pants, of the pre-fastened type normally referred to as "training pants" or "pull-ups" consists of producing a composite tape formed of a continuous chain of product blanks, aligned in order to have the transverse axis of each single absorbent article coincident with the direction of longitudinal advancing of the tape itself.

The continuous composite tape is folded in two around a longitudinal axis coaxial with said transverse axis of each single absorbent article, so that the longitudinal end portions of the composite tape overlap with each other.

The two edges of the composite tape overlapping in this way are connected together with transverse weld lines, so as to define the waist and leg openings of each absorbent article. The continuous chain of blanks is then subjected to a cutting operation, at the welds, to divide the finished products from each other.

This particular manufacturing methodology is normally identified by the term "Cross-Direction" (or CD), distinguishing it from the more traditional form of production, said "Machine Direction" (or MD), which forms the absorbent articles while they travel with their longitudinal axis parallel to the direction of production.

An example of a method for manufacturing absorbent sanitary products of the CD type is described in the documents EP-A-1 013 251, IT-0001379452, TO2012A000209 and TO2012A000210 by the same applicant.

In the "Cross Direction" methods, it is necessary to ensure a correct positioning of the transverse welds of each product, in order to correctly define the waist and leg openings of each single diaper. Furthermore, it is important that the cutting line intended to divide the individual products from each other is correctly positioned at the welds produced between two adjacent absorbent products and, very importantly, both the welds and the cuts should present a constant angular position with respect to the advancing direction of the sheet, or, in other words, they must always present, at least the welds, the same angular relation with respect to the edges of the composite tape.

As well known to the skilled person, producing the transverse welding lines on the composite tape currently constitutes one of the major technological limitations to the operation speed of the production lines.

Indeed, the welding system must guarantee an adequate sealing force of the transverse welds that produce the closure of the diaper around the user's waist.

In order to guarantee a resistance that allows the safe use of the absorbent article, the side welds must typically present values of tensile strength of the order of 1000 grams per inch (2.54 cm).

The apparatuses that are used for producing transverse welds are, typically, welding devices that use ultrasonic equipment that provides the energy to carry out the welding of the composite tape with a forging-type process. In practice, the welding is carried out by repeatedly striking, with a strong intensity, the layers of material interposed between the vibrating device of the ultrasonic system and the respective counter-roller, which is provided with a series of protuberances (or welding pattern), where the material of the tape is repeatedly pressed by the blows inflicted by the vibrating element of the ultrasonic system.

Due to the repeated blows, the material of the tape interposed between the welding pattern and the vibrating element of the ultrasonic system decreases in thickness by compressing and moving toward the side edges of the protuberances of the counter-roller, generating the heat necessary to melt all the layers of material.

In order to obtain an acceptable weld, it is necessary to produce a minimum number of blows that, for each composite tape, may vary according to the type of material, the number of layers and the configuration of the welding pattern. Furthermore, increasing the speed with which the tape passes through the welding system decreases the number of blows that can be inflicted to the tape by an ultrasonic vibrating system at a given frequency, thereby negatively affecting the quality of the weld.

EP-A-1393701 discloses a method and an apparatus for producing a wearing article according to the preamble of claims 1 and 6.

### Object and summary

The object of the present invention is to provide a method and an apparatus for producing transverse weld lines on a composite tape, which can be used for producing sanitary articles wearable in the manner of pants of the pre-fastened type, which is also successfully usable in production lines operating at high production speeds.

According to the present invention, this object is achieved by a method forming the subject of claim 1 and by an apparatus according to claim 6.

The claims form an integral part of the disclosure provided in relation to the invention.

### Brief description of the drawings

The invention will now be described, purely by way of non-limiting example, with reference to the accompanying figures, in which:
- Figure 1 is a perspective view of an absorbent product of the pre-fastened pant type producible with the apparatus according to a preferred embodiment of the present invention, in the closed configuration and ready for use,
- Figure 2 is a schematic perspective view of the absorbent product of Figure 1 in the open extended configuration,
- Figures 3a and 3b represent the composite tapes formed of the semi-finished blanks of two types of absorbent products of the pre-fastened pant type producible with the apparatus according to a preferred embodiment of the present invention,
- Figure 4 is a schematic perspective view of the production steps of an absorbent article according to a preferred embodiment of the present invention,
- Figure 5 is a schematic view of a braking device of the present invention according to a preferred embodiment, and
- Figure 6 is a schematic perspective view of the production steps of an absorbent article according to a further preferred embodiment of the present invention.

### Detailed description of the embodiments

Below, a manufacturing method of an absorbent article **10** will be described according to a preferred embodiment of the present invention.

The references used herein are for convenience only and therefore do not define the field of protection or the scope of the embodiments, therefore, the definitions "front" or "rear" referring to the absorbent sanitary article only depend on the end use of the product itself, or rather as it is worn by the user, while the definitions "upper" or "lower" relative to any elements present in one or more embodiments described only depend on the point of view of the observer, with respect to the drawings attached to this document without, therefore, limiting the principle and/or the scope of the invention.

Note that in the following description of the drawings, elements similar to each other will be designated with the same reference numeral.

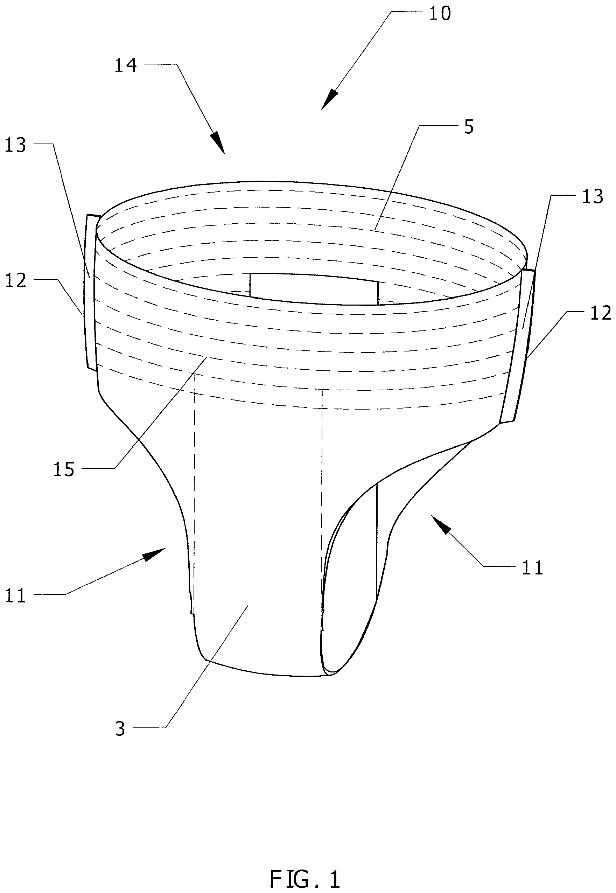

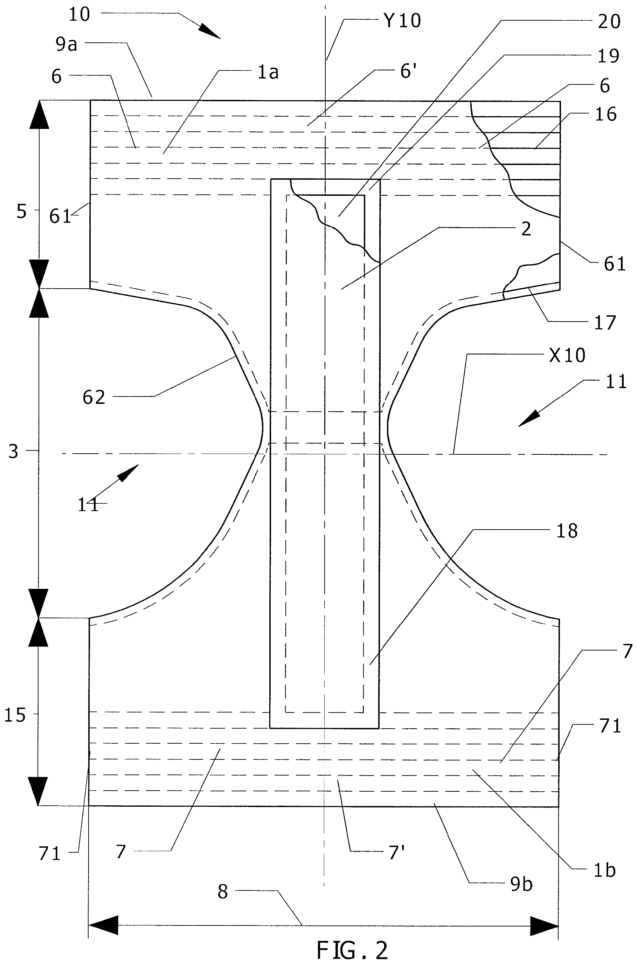

In Figures **1** and **2****,** a sanitary article **10** is represented, which can be produced with a method and an apparatus according to the disclosures provided by the present invention.

In Figure **1****,** the sanitary article **10** is represented in its closed condition, where it presents a conformation essentially comparable to pants. Instead, Figure **2** shows the same product **10** in the open and extended configuration, in which it is possible to identify a first waist region **5,** a second waist region **15** and a crotch region **3** located between the two waist regions **5** and **15,** as well as a pair of mutually perpendicular axes, longitudinal **Y10** and transversal **X10.**

In the first waist region **5,** it is possible to identify a pair of first side panels **6** separated by a first central waist region **6'** interposed between them. Similarly, in the second waist region **15,** a second central waist region **7'** can be identified that separates the second waist panels **7.** On each of the side panels it is possible to identify a distal edge **61** for the first side panels **6** and **71** for the second side panels **7.**

The absorbent article **10** is folded around its transverse axis **X10** to assume its pant-configuration so that the two waist regions overlap with each other in order to be able to carry out the transverse welds **13** that join together the distal edges **61, 71** of the side panels **6, 7** of the diaper and that confer the characteristic pant-conformation to it, defining the opening **14** for the waist and the two openings **11** for the legs, as shown in Figure **1****.**

The first waist region **5** and the second waist region **15** are typically elasticated, for example with elastic elements **16** in the form of threads. The two waist regions **5, 15** are interconnected by the crotch region **3** in which, typically, an absorbent insert **2** is present.

The first and the second waist regions **5, 15** are elasticated as they are used to maintain the absorbent insert **2** adherent to the user's body in a comfortable manner, even when it becomes heavy after having absorbed body fluids.

Diapers of this type can have different configurations from each other, both regarding the absorbent insert **2,** and regarding the first and second waist regions **5** and **15.**

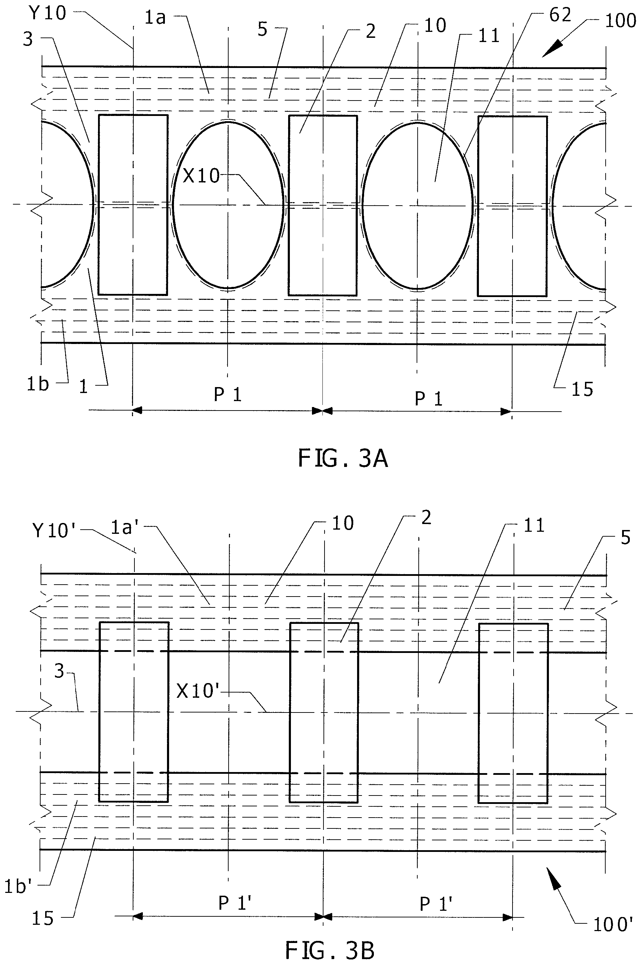

In Figures **3a** and **3b****,** two embodiments are represented for producing sanitary articles **10** and **10',** by way of example, well known to the skilled person. The continuous tapes formed of semi-finished blanks illustrated in Figures **3a** and **3b** have different structures from each other, but both can be obtained using the method of the present invention.

In Figure **3a****,** the absorbent article **10** presents the first waist region **5,** the second waist region **15** and the crotch region **3,** produced with a single tape **1,** on which, typically, a series of cuts **62** are made, of appropriate shape, each of which contributes to define the contour of the leg openings **11** of two consecutive sanitary articles **10.** The absorbent insert **2** is typically placed between two consecutive cuts **62.**

In the absorbent article **10'** obtainable by the chain of blanks of Figure **3b****,** the first waist region **5** and the second waist region **15** are formed of two tapes **1a'** and **1b'** separate and parallel to each other, while the absorbent insert **2** joins the two tapes **1a'** and **1b'** to each other, and forms the crotch region **3** and the opening for the legs **11.**

For both of the absorbent articles **10** and **10',** the absorbent insert **2** presents a structure in which the following features are recognizable (in addition to various other accessory elements), as illustrated in Figure **2****,** which represents, in particular, an article 10 obtainable by the chain of blanks of Figure **3a****:**
- an upper layer or topsheet **18** permeable to evacuated body fluids, intended to be facing the user's body;
- a lower layer or backsheet **19** impermeable to body fluids; and
- an absorbent core **20** interposed between the topsheet **18** and the backsheet **19** for absorbing and retaining the exudates.

On the absorbent product **10,** and in particular on the absorbent insert **2,** other elements may be present that contribute to increase the characteristics of wearability and absorbency, all well known to persons skilled in the art and of which extensive documentation is available.

The absorbent insert **2** and the tape **1,** as illustrated in Figure **3a****,** can be made integral with each other by means of strips of adhesive and/or by means of welds produced with any system known per se, such as for example thermo-mechanical or ultrasonic welding.

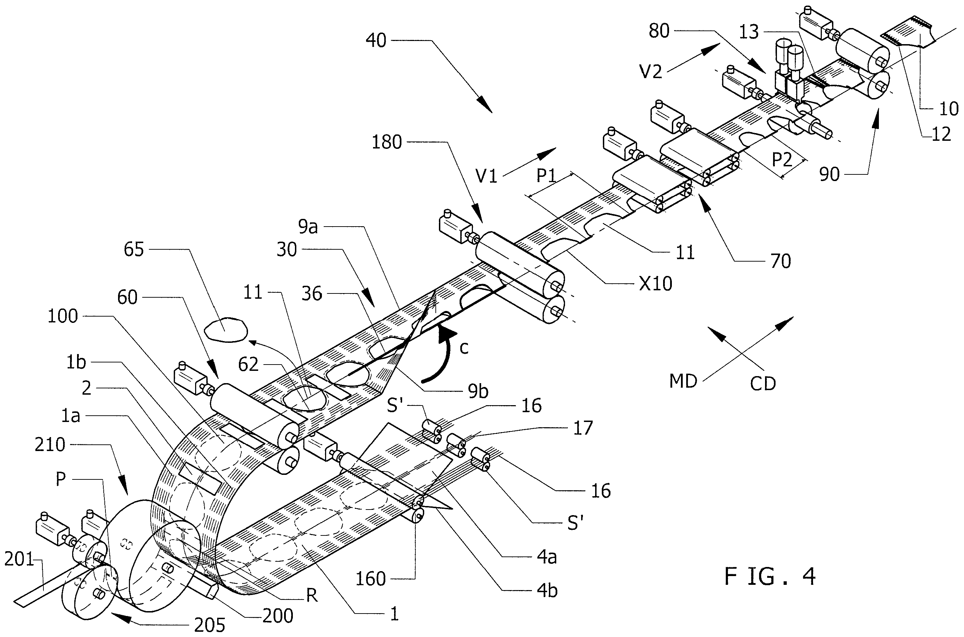

As already mentioned, the two types of sanitary articles **10** and **10',** although having structural differences, can both be produced by the manufacturing method **40** according to a first preferred embodiment illustrated in Figure **4****.**

Below, therefore, apart from specific references, the method **40** will be described of the embodiment illustrated in Figure **4** with reference to the sanitary article **10** illustrated in Figures **1****,** **2** and **3a****.**

In Figure **4****,** a manufacturing method **40** is schematically shown according to a preferred embodiment of the invention, in which the production steps of the composite tape of elastic material **1** and of the relative sanitary articles **10** are represented.

In the manufacturing method **40** according to the preferred embodiment illustrated in Figure **4****,** it is possible to identify two main directions **MD** and **CD** perpendicular to each other, wherein the first direction **MD** is parallel to the production line flow.

In the preferred embodiment of Figure **4****,** the manufacturing method **40** of the absorbent article **10,** includes a first step in which the tape **1** is produced.

A first and a second sheet **4a, 4b** and the appropriately tensioned elastic elements **16** are joined together in a fixing station **160.** The first sheet **4a** and the second sheet **4b,** in combination with the elastic elements **16,** form a first elastic structure **1a** and a second elastic structure **1b** at the longitudinal end portions of the tape **1,** each of which contributes to form, respectively, the first waist region **5** and the second waist region **15** of the absorbent article **10.**

**T**he inner sheet **4a** and the outer sheet **4b** are each supplied to the fixing station **160** by a respective unwinding source, not shown in Figure **4** but of a type known in the art, such as, for example, limiting ourselves to patent documents of the applicant, EP 1 013 585 A1.

The elastic elements **16** can be also conveyed to the fixing station **160** from a feed system **S',** of known type, such as, for example, the unwinder for elastic materials on a reel described in US 6676054.

Typically, both the inner sheet **4a** and the outer sheet **4b** are supplied to the welding station **160** at a first speed **V1.**

As is well known to the skilled person, the first speed **V1** is equal to the transverse width 8 of the absorbent article **10** in an open and extended condition (as represented in Figure **2** or in Figures **3a** and **3b**) multiplied by the number N of absorbent articles that the production line is able to produce in the unit of time (1 second).

The elastic threads **16** used to produce the elastic structures **1a** and **1b** (forming the first and the second waist regions **5, 15** of the absorbent article **10**) are fed to the fixing station **160** with a speed **V16,** less than said speed **V1.**

Normally, the two speeds **V1** and **V16** are very different from each other. Typically, the feeding speed **V16** of the elastic elements **16** is about half of the first feeding speed **V1** of the sheets **4a** and **4b.** The difference in speed between the sheets **4a** and **4b** and the elastic threads **16** causes an elongation of the elastic threads **16** proportional to the difference in speed. In this way, the required characteristics of elasticity and tension are conferred to the elastic structures **1a** and **1b.** The first elastic structure **1a** and the second elastic structure **1b** of the tape **1** (produced by lamination of the inner sheet **4a,** the outer sheet **4b** and the elastic elements **16**) present a state of tension **T1** proportional to the difference in speed **(V1, V16)** of their component elements.

Apparatuses and methods suitable for performing the functions of the fixing unit **160** are well known in the art and may use various technologies using adhesive or welding systems that can be implemented with heated rollers or with ultrasound apparatuses. Limiting ourselves to technologies that use application systems of hot-melt adhesives, the latter can be applied on one or both sheets or directly onto the elastic elements, or, alternatively, both on the sheets and on the elastic elements.

The welding unit **160** is typically provided with a pair of counter-rotating motorized rollers that press against each other, in order to adhere together all the materials passing through it and that make up the elasticated structures **1a** and **1b.** Typically, each of said rollers can be provided with a non-stick treatment to reduce contamination from the adhesive, if used in the manufacturing process.

To release the state of tension **T1** of the elastic structures **1a** and **1b,** as well known to the skilled person, the elastic elements **16** must return to their resting condition, or rather, must return to a length corresponding to the feeding speed **V16,** which is typically half of the feeding speed **V1** of the two sheets **4a** and **4b.** In other words, to cancel the tension **T1,** the elastic elements **16** must retract and return to their original length, approximately equal to half of the transverse width **8** of the absorbent article **10** in an open and extended condition. In this way, the elastic structures **1a** and **1b** retract, forming the characteristic "pleating" due precisely to the fact that said elastic elements **16** and said sheets **4a** and **4b** are joined together.

The fixing unit **160** is also able to perform the function of the control station of the feeding speed of the tape **1** (and therefore of the elastic structures **1a** and **1b**) toward all other processing stations downstream of it.

As illustrated in Figure **4****,** in the preferred embodiment, during the lamination step of the inner sheet **4a** and the outer sheet **4b** with the elastic elements **16,** it is possible to join further elastic elements **17** to said sheets, suitable for elasticizing the leg openings **11** so as to confer greater wearability to the absorbent article **10.**

Methods and apparatuses for the application of elastic elements suitable for improving the wearability of the absorbent article **10** conferring an elastic structure at the opening of the legs **11** are well known in the art. An example of equipment and method suitable for carrying out an application of elastic elements of this type is described in EP 1 842 516 A1 by the same Applicant.

In the preferred embodiment illustrated in Figures **1****,** **2** and **3a****,** the two sheets **4a, 4b** can be of nonwoven fabric, which may have undergone treatments that render them water-repellent, so as to contain any loss of liquids and avoid any possible contamination of the user's clothing with which the sanitary article **10** may come into contact.

The elastic elements **16** and **17** can be selected from several materials available on the market, such as strips of synthetic rubber produced by Fulflex or Lycra® threads produced by Invista. Otherwise, on the market there is a wide range of natural or synthetic materials, able to be conveniently used to produce an absorbent article **10.**

In the preferred embodiment of Figure **4****,** after having produced the tape 1, i.e. the elastic structures **1a** and **1b,** the device **20** applies absorbent inserts **2** onto it in order to produce a composite tape **100** formed of a continuous chain of product blanks aligned so as to have the transverse axis **X10** of each absorbent article **10** parallel to the **MD** direction of the production line flow.

The absorbent inserts **2** are typically produced according to a method called "Machine Direction", i.e. with the longitudinal axis of the insert parallel to the **MD** direction of the production line flow.

The tape **201** of blanks of absorbent inserts **2** is typically fed to a cutting unit **205,** which segments it so as to produce the individual absorbent inserts **2,** and then to a Turn and Repitch device **210** which picks up each of said absorbent inserts **2** directly from the cutting unit **205** at a gripping point **P** and changes their orientation by rotating them by **90°** in order to make the transversal axis **X2** parallel to the production direction **MD** of the machine.

The Turn and Repitch device **210,** prior to depositing the absorbent insert **2** on the tape **1** at the release point **R,** between the first elastic structure **1a** and the second elastic structure **1b,** synchronizes the speed of each absorbent insert **2,** with the speed **V1** of the tape **1.**

Before the release point **R,** a device for applying adhesive **200** is typically inserted on the tape **1,** which spreads a film of glue, of suitable size and amount, in order to join the tape **1** together, and in particular the first elastic structure **1a** and the second elastic structure **1b** with the absorbent inserts **2.**

In this way, a composite tape **100** is produced, formed of a tape **1,** which comprises a first elastic structure **1a,** and a second elastic structure **1b** on which a sequence of absorbent inserts **2** is applied, spaced apart at a first constant pitch **P1,** and with the respective transverse axis **X2** coinciding with the transverse axis **X10** of the corresponding absorbent article **10** and parallel to the **MD** direction of the production line flow.

An example of a Turn and Repitch device **210** suitable for use in the method of the present invention is described in EP 1 947 037 A1 by the same Applicant.

The speed **V1** of the tape **1** and the pitch **P1** at which the absorbent inserts **2** are positioned are directly proportional to each other. In fact, the speed **V1** expressed in meters per second, is given by the pitch **P1** expressed in meters multiplied by the number of absorbent articles **10** that the production line is able to produce in the unit of time, typically 1 second. In other words, the pitch **P1** is equal to the transverse width **8** of the absorbent article **10** in an open and extended condition.

In the preferred embodiment, as illustrated in Figure **4****,** on the tape **1,** between the first elastic structure **1a** and the second elastic structure **1b,** at the crotch region **3** on which the absorbent insert **2** is typically arranged, a series of cuts **6** of appropriate shape can be made, by means of a shaped cutting unit **60.** Each of the cuts **62** removes a portion of material **65** from the elasticated tape **1,** and defines the contour of the leg openings **11** of two consecutive sanitary articles **10** which, in the preferred embodiment illustrated in Figures **3a** and **4****,** can be circumscribed by the elastic elements **17** for the legs.

The shaped cutting unit **60** in the production flow can be placed either downstream or upstream of the Turn and Repitch device **210.** For example, contrary to what is shown in Figure **4****,** the shaped cutting unit **60** can be placed upstream of the Turn and Repitch unit **210** to avoid possible interference of the cutting blade of the unit **60** with the absorbent insert **2.**

In the manufacturing method **40** of the absorbent sanitary article **10,** according to the preferred embodiment illustrated in Figure **4****,** once the composite tape **100** is produced, consisting of a chain of semi-finished blanks of absorbent sanitary articles **10,** the first elastic structure **1a** and the second elastic structure **1b** are overlapped on each other by folding the composite tape **100** longitudinally around a rod **36** of a longitudinal folding station **30.** The bar **36** is arranged parallel to the direction of longitudinal advancing **MD** of the production line flow, and typically coinciding with the axis of symmetry **X10** of the individual absorbent products **10,** in order to bring together the longitudinal edges **9a** and **9b** of the composite tape **100.**

In Figure **4****,** the arrow **C** schematically indicates the folding movement which achieves the overlapping of the first elastic structure **1a** with the second elastic structure **1b** and of the respective aforesaid longitudinal edges **9a** and **9b.**

An example of equipment and method **30** suitable for carrying out a fold of this type is described in the application TO2011A001085 by the same Applicant.

As illustrated in the preferred embodiment of Figure **4****,** in the device **40,** the composite tape **100** passes through the longitudinal folding device **30** that carries out the overlapping of the first elastic structure **1a** with the second elastic structure **1b,** at the first speed **V1.** Homologous elements of consecutive absorbent sanitary articles **10,** at this step of the process, are spaced apart by a first pitch **P1.**

Subsequently, after said overlapping operation of the first and second elastic structures **1a, 1b,** the composite tape **100** is fed to a braking station **70** which reduces the speed by taking it from the first speed **V1** to a second speed **V2,** typically between said first speed **V1** of the tape **100** and the feeding speed **V16** of the elastic elements **16.** In this way, at the outlet of the braking station **70,** the blanks of the consecutive absorbent sanitary articles **10,** which make up the composite tape **100,** slow down to said second speed **V2** and are spaced apart by a second pitch **P2.**

The reduction in speed is made possible because the state of tension **T1** of the composite tape **100** is partially released at the braking station **70** by bringing the elastic elements **16** closer to their rest condition. This involves the reduction of the distance between two consecutive products, which will have a second pitch **P2** less than the first pitch **P1** in the composite tape **100.**

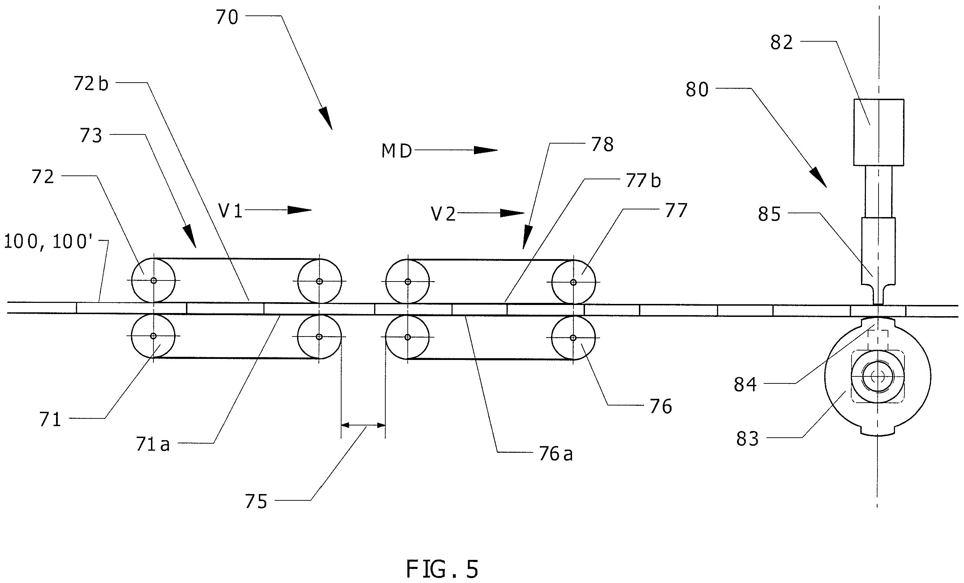

In the preferred embodiment illustrated in Figure **4****,** a particularly advantageous device **70** is represented to implement the braking of the tape **100,** illustrated in greater detail in Figure **5****.**

With reference to Figure **5****,** the brake section **70** comprises a first pair of motorized conveyor belts **73,** formed from a lower conveyor belt **71** and an upper conveyor belt **72,** and a second pair of motorized conveyor belts **78,** in turn formed from a lower conveyor belt **76** and an upper conveyor belt **77.**

The lower conveyor belts **71** and **76** and the upper conveyor belts **72** and **77** of the conveyors **73** and **78** forming the braking device **70** are arranged, typically, on opposite sides of the tape **100** folded longitudinally. Therefore, each conveyor belt is in contact with a respective elastic structure **1a** and **1b.**

In a preferred embodiment, as is more evident in the view of Figure **5****,** in the braking device **70,** the branches **71a, 72b** and **76a, 77b** of the conveyor belts **73** and **78,** can be connected to a source of sub-atmospheric pressure in order to be able to carry out the gripping of the respective elastic structures **1a** and **1b** of the composite tape **100,** for suction as well as pressure.

In the preferred embodiment, as illustrated in Figure **5****,** in the first conveyor belt **73,** the branches **71a** and **72b** move in the direction **MD** at the first speed **V1,** while in the second conveyor belt **78,** the branches **76a** and **77b** move in the direction **MD** at the second speed **V2.**

In the transition zone **75** between the first conveyor belt **73,** which moves at the first speed **V1** and the second conveyor belt **78,** which moves at the second speed **V2,** the tape **100** is left free to release the tension **T1** corresponding to the first speed **V1,** and slows down to the second speed **V2,** which corresponds to a second state of tension **T2** less than **T1.**

The speed **V2** of the composite tape **100,** at the outlet of the braking station **70** is preferably greater than the speed **V16** of the elastic elements **16** for the following reasons:
- to obtain a weld of acceptable quality, in effect, when the laminated tape **1** releases the tension **T1,** it will retract forming the characteristic "pleating" due to the fact that said elastic threads are integrally joined to the inner and outer sheets, creating, therefore, transverse wrinkles. The abovesaid pleating could prove detrimental to the process of downstream transverse welding - that will be described below - causing a non-uniform increase in thickness of material between the welding elements;
- to control the moving composite tape **100,** it is necessary to maintain a minimum value of the longitudinal tension.

In the preferred embodiment of Figure **4****,** an advantageous speed **V2** for the method of the present invention is in the range that can vary between a minimum value of **0.7 V1** and a maximum value of **0.9 V1.**

After slowing down the composite tape **100** formed of the continuous chain of product blanks to the second speed **V2,** the latter is processed by the welding device **80** suitable for producing the transverse welds **13,** which join together the first elastic structure **1a** with the second elastic structure **1b,** contributing to confer the characteristic pant-conformation to the absorbent article **10,** defining the waist opening **14** and the two leg openings **11.**

In the preferred embodiment illustrated in Figure **4** and in Figure **5****,** the welding operation is carried out by the equipment **80,** which typically consists of an ultrasonic welding device **82** which cooperates with a counter-roller **83.**

Typically, the tape **100** of blanks fed at the second speed **V2** is passed through the welding apparatus **80** between the ultrasonic welding device **82** and the counter-roller **83.**

The counter-roller **83** rotates in order to have the respective peripheral speed essentially identical to the advancing speed **V2** of the composite tape **100** formed of the chain of blanks of sanitary articles **10.**

An example of a method and equipment **80** suitable to carry out a transverse weld **13** of this type is described in WO 2010/101287 A1 by Yamamoto.

In the welding device described here, the vibrating apparatus of the ultrasonic system **82,** which provides the energy to create the weld **13** of the first and second elastic structures **1a** and **1b** implements, as already said, a process of the forging type. In practice, in the apparatus **80,** the welds **13** are produced by the vibrating device of the ultrasonic system **82,** which repeatedly strikes, with a strong intensity, the layers of material interposed between it and the respective counter-roller **83,** which is typically provided on its outer surface with appropriate welding blades **84** protruding transversely to the feed direction of the composite tape **100.**

Due to the repeated blows, the material of the tape **100** interposed between the welding pattern and the vibrating element **85** of the ultrasonic system **82** is pressed, compressed, reduced in thickness and forced to migrate toward the side edges of the welding blades **84** of the counter-roller **83,** generating the heat necessary to melt all the layers of material. An example of a counter-roller **83** provided with welding blades and suitable to carry out transverse welding lines **13** is described in TO2012A000210 by the same Applicant.

It is necessary to apply a minimum number of blows to the elastic structures **1a** and **1b** in order to obtain an acceptable weld, which typically varies according to the type of elastic structures **1a, 1b** of the composite tape **100,** the material, the number of layers and the configuration of the welding pattern.

The reduction of the crossing speed of the welding unit **80** by the composite tape **100** formed of the chain of blanks of sanitary articles **10** to the second speed **V2,** carried out by the braking device **70,** allows the ultrasonic system, vibrating at a given frequency, to confer a greater number of blows to the composite tape **100** with respect to a line that processes a composite tape **100** at the first speed **V1.**

After making the transverse welds **13,** the composite tape **100** is sent, at the second speed **V2,** to the cutting station **90,** which segments it into a plurality of sanitary articles **10.**

An example of a method and device **90** suitable to carry out the cutting operation in production lines of this type is described in TO2012A000209 by the same Applicant.

In the preferred embodiment, as shown in Figure **4****,** immediately after the longitudinal folding unit **30** and before the braking unit **70,** it is possible to insert an additional welding device **180** that carries out technical welding, which can be, for example, welding points.

Said technical welds, which are designed to break easily at the time in which the absorbent sanitary article **10** is worn, have the function of maintaining the two elastic structures **1a** and **1b** integral with each other during the operations of deceleration of the composite tape **100** thus avoiding relative movements between the two elastic structures.

Devices suitable for carrying out technical welding for the aforesaid object are well known in the art, such as mechanical welding systems with heated rotating rollers, as represented in Figure **4****,** or ultrasonic welding devices. Examples of welding equipment **180** suitable for carrying out the technical welding as described above are described in US 4,919,738 A and US 4,854,984 A both by Ball et al.

The composite tape **100'** of Figure **3b** can be processed in the apparatus of Figure **4** in an analogous manner to that described for the composite tape **100** of Figure **3a****,** except for the fact that the two elastic structures **1a'** and **1b'** are produced independently from each other and joined by the inserts **2** so as to form a single tape **100'.**

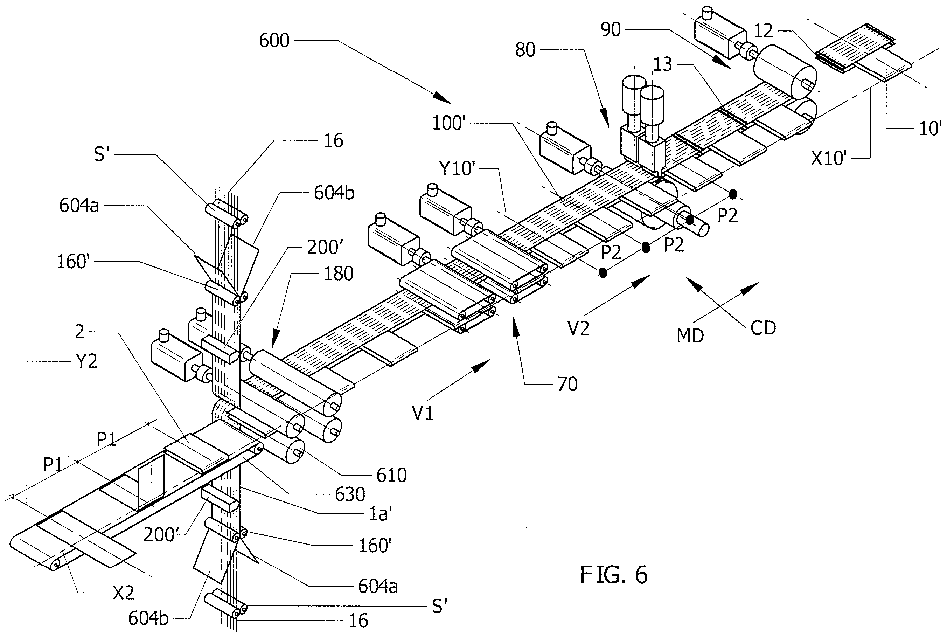

A further preferred embodiment of the present invention can be implemented, as illustrated in Figure **6****,** in the particularly advantageous method **600** for being able to produce an absorbent sanitary article **10',** as schematically represented in Figure **3b****,** in which the first waist region **5** and the second waist region **15** are formed of two separate tapes parallel to each other and formed, respectively, by a first elastic structure **1a'** and by a second elastic structure **1b',** and where the absorbent insert **2,** joining together the two elastic structures **1a'** and **1b',** contributes in forming the crotch region **3** and the leg openings **11.**

For a better understanding, the elements and/or devices common to both the method **40** of Figure 4 and the method **600** of Figure **6** will be identified by the same numbers.

In the further preferred embodiment of Figure **6****,** the manufacturing method **600** of the absorbent sanitary article **10',** includes a first step in which the two elastic structures **1a'** and **1b'** are produced. Each of the two elastic structures **1a'** and **1b'** can be produced by joining together a first sheet or inner sheet **604a,** and a second sheet or outer sheet **604b** with the elastic elements **16** interposed between said sheets.

The first and the second sheets **604a, 604b** and the elastic elements **16,** belonging to each of the two elastic structures **1a'** and **1b',** are joined together in respective fixing stations **160'.**

As highlighted in Figure **6****,** the first elastic structure **1a'** and the second elastic structure **1b'** contribute to form, respectively, the first waist region **5** and the second waist region **15** of the absorbent sanitary article **10'.**

In this case as well, the inner sheets **604a** and the outer sheets **604b** are supplied to the welding station **160'** each from their own unwinding source, not illustrated in the figures but of a known type in the art.

Analogously, the elastic elements **16** conveyed to the respective fixing station **160'** can come from a feed system **S',** also of known type.

In the embodiment illustrated in Figure **6****,** each of the inner sheets **604a** and outer sheets **604b,** can also be fed in this case to the respective fixing station **160'** at a first speed **V1.**

The elastic threads **16,** which form the first elastic structure **1a'** and the second elastic structure **1b'** that provide the elasticization of the first and the second waist regions **5, 15** of the absorbent sanitary article **10',** are fed to the respective welding station **160'** with a speed **V16,** less than said speed **V1** of the respective sheets **604a, 604b.**

In this case as well, the two speeds **V1** and **V16** can be very different from each other. Typically, the feeding speed **V16** of the elastic elements **16** can be about half of the first feeding speed **V1** of the respective sheets **604a, 604b.** Said difference in speed is compensated, during the lamination step, by an elongation of the elastic threads **16** proportional to said difference in speed, in order to have elastic structures **1a'** and **1b'** with appropriate characteristics of elasticity and tension **T1.**

In the embodiment illustrated in Figure **6****,** the fixing unit **160',** suitable for integrally and permanently combining the sheets **604a** and **604b** and the elastic threads **16,** may be entirely similar to the fixing unit **160** described in the embodiment of Figure **4****.** Each of the fixing units **160'** may be equipped with a pair of counter-rotating rollers that press against each other, in order to adhere together all materials that pass through it, and which contribute to form the respective elasticated structures **1a', 1b'.** Typically, each of the said rollers may be provided with a non-stick treatment on their outer surface to reduce contamination by the adhesive, if used in the manufacturing process.

The absorbent inserts **2,** in this further preferred embodiment illustrated in Figure **6****,** can also be produced according to a method known as "Machine Direction", so as to have a tape **201** of blanks of absorbent inserts **2** parallel to the MD direction of the production line flow. Typically, the tape **201** is segmented by a cutting unit (not shown in Figure **6**), in order to produce a flow of individual absorbent inserts **2.**

Each absorbent insert **2,** analogously to that described in the previous embodiment of Figure **4****,** may be picked up by a Turn and Repitch device (not shown in Figure **6**) that synchronizes the speed with the speed **V1** of the elastic structures **1a'** and **1b'** and changes its orientation by rotating it by **90°** in order to have the respective transversal axis **X2** parallel to the production direction **MD** of the machine and coinciding with the axis **X10'** of the sanitary article **10',** so as to create a plurality of absorbent inserts equidistant from each other and spaced at a constant pitch **P1.**

Unlike the embodiment of Figure **4****,** in the further preferred embodiment illustrated in Figure **6****,** the individual absorbent inserts **2** released from the Turn and Repitch device, instead of being deposited directly on the elastic structures **1a'** and **1b',** are released onto a longitudinal folding device **630** that folds each absorbent insert **2** in two, around the respective transverse axis **X2.**

An example of the method and the device **630** capable of carrying out the folding operation of the inserts **2** in production lines of this type is described in TO2010A000143 by the same Applicant.

In the embodiment of Figure **6****,** after having produced the first elastic structures **1a'** and the second elastic structure **1b',** these are sent to the additional fixing equipment **610** where the elastic structures **1a'** and **1b'** are joined to the absorbent inserts **2** already folded in two, typically by means of adhesive applied to each elastic structure **1a'** and **1b'** with respective application devices **200',** which spread layers of adhesive of appropriate size and quantity at the position of the relative absorbent inserts **2,** in order to produce a composite tape **100'** formed of a continuous chain of blanks of sanitary articles **10'.**

From this moment on, the composite tape **100'** in the preferred embodiment of Figure **6** is treated in the same way in which the homologous composite tape **100** is treated in the method illustrated in Figure **4****.** Therefore, the composite tape **100'** is firstly decelerated by the braking device **70** to the second speed **V2,** less than the first speed **V1,** but greater than the speed **V16,** and subsequently the welds **13** are made on the composite tape **100',** which join together the first elastic structure **1a'** and the second overlapping elastic structure **1b',** contributing to confer the characteristic pant-conformation to the absorbent article **10',** defining the waist opening **14** and the two leg openings **11.**

## Claims

1. A method for the transverse welding of a composite tape **(100, 100')** comprising the steps of:
- forming a composite tape **(100, 100')** comprising a first and a second elastic structure **(1a, 1a', 1b, 1b')** overlapping each other, which is continuously advanced in a longitudinal direction (**MD**) at a first feeding speed (**V1**) and at a first feed tension (**T1**);
- slowing down (**70**) said composite tape (**100**), taking it to a second speed (**V2**) and a second tension (**T2**), respectively less than said first feeding speed (**V1**) and said first feed tension (**T1**),
- feeding said composite tape **(100, 100')** at said second speed (**V2**) and tension (**T2**) to a welding station (**80**) and welding (**80**) said composite tape (**100, 100'**) with welding lines (**13**) transverse to said longitudinal feed direction (**MD**), and
- feeding said composite tape **(100, 100')** at said second speed (**V2**) and tension (**T2**) to a cutting station **(90)** and cutting (**90**) said composite tape (**100, 100'**) with cuts (**12**) transverse to said longitudinal feed direction (**MD**),
**characterized in that** said slowing down step of said composite tape **(100, 100')** is carried out by feeding the said composite tape **(100, 100')** to a first motorized belt conveyor (**73**) traveling at said first speed (**V1**) and subsequently to a second motorized belt conveyor (**78**) traveling at said second speed (**V2**), wherein said first and second belt conveyors (**73, 78**) are spaced apart in the longitudinal direction.

2. A method according to claim 1, wherein each of said elastic structures (**1a, 1a', 1b, 1b'**) comprises a first web material (**4a**) and a second web material (**4b**) with interposed elastic elements (**16**).

3. A method according to any one of the preceding claims, wherein the two elastic structures (**1a, 1b**) are longitudinal end portions of a single tape (1, **100**), wherein said elastic structures (**1a, 1b**) overlap each other by means of folding (**30**) said single tape (**100**) along its longitudinal axis (**X10**).

4. A method according to claims 1 or 2, wherein the two elastic structures (**1a', 1b'**) are transversely joined by discrete elements (**2**) equidistant (**P1'**) to each other and folded around a transverse axis (**X2**), wherein the method comprises the step of forming a composite tape structure (**100'**), wherein said elastic structures (**1a', 1b'**) are applied one on top of the other with portions of said discrete elements (**2**) arranged between said folded elastic structures (**1a', 1b'**).

5. A method according to any one of the preceding claims, comprising a technical welding step (**180**) of said overlapping (**30, 610**) elastic structures (**1a, 1a', 1b, 1b'**) before said slowing down step (**70**).

6. Apparatus for carrying out the method of any one of the preceding claims, said apparatus comprising:
- a braking device (**70**) adapted to slow down said composite tape (**100, 100'**) from a first speed (**V1**) to a second speed (**V2**)**,** and
- a welding device (**80**) adapted to produce welding lines (**13**) transverse to said longitudinal feed direction (**MD**) on said composite tape (**100, 100'**) traveling at said second speed (**V2**),
**characterised in that** said braking device (70) comprises a first motorized belt conveyor (73) traveling at said first speed (V1) and a second motorized belt conveyor (78) traveling at said second speed (V2), wherein said first and second belt conveyors (73, 78) are spaced apart in the longitudinal direction.

7. Apparatus according to claim 6, comprising a welding device **(180)** adapted to form technical welds on said first and second overlapping elastic structures **(1a, 1a', 1b, 1b')** on said composite tape **(100, 100')** traveling at said first speed (**V1**)**,** said welding device (**180**) being located upstream of said braking device (**70**).

## Patentansprüche

1. Verfahren zum Querschweißen eines Verbundbandes (**100, 100'**), umfassend die folgenden Schritte:
- Bilden eines Verbundbandes (**100, 100'**), das eine erste und eine zweite elastische Struktur **(1a, 1a', 1b, 1b')** umfasst, die einander überlappen, und das in einer Längsrichtung (**MD**) mit einer ersten Zuführgeschwindigkeit (**V1**) und einer ersten Zuführspannung (**T1**) kontinuierlich vorwärtsbewegt wird;
- Verlangsamen (**70**) des Verbundbandes (**100**), wodurch dieses auf eine zweite Geschwindigkeit (**V2**) und eine zweite Spannung (**T2**) gebracht wird, die geringer sind als die erste Zuführgeschwindigkeit (**V1**) bzw. die erste Zuführspannung (**T1**),
- Zuführen des Verbundbandes (**100, 100'**) mit der zweiten Geschwindigkeit (**V2**) und Spannung (**T2**) zu einer Schweißstation (**80**) und Verschweißen (**80**) des Verbundbandes (**100, 100'**) mit quer zu der Längszuführrichtung (**MD**) verlaufenden Schweißlinien (**13**), und
- Zuführen des Verbundbandes (**100, 100'**) mit der zweiten Geschwindigkeit (**V2**) und Spannung (**T2**) zu einer Schneidestation (**90**) und Schneiden (**90**) des Verbundbandes (**100, 100'**) mit quer zu der Längszuführrichtung (**MD**) verlaufenden Schnitten (**12**),
**dadurch gekennzeichnet, dass** der Schritt des Verlangsamens des Verbundbandes (**100, 100'**) ausgeführt wird durch Zuführen des Verbundbandes (**100, 100'**) zu einem sich mit der ersten Geschwindigkeit (**V1**) bewegenden ersten motorisierten Bandförderer (**73**) und anschließend zu einem sich mit der zweiten Geschwindigkeit (**V2**) bewegenden zweiten motorisierten Bandförderer (**78**), wobei der erste und der zweite Bandförderer (**73, 78**) in Längsrichtung beabstandet sind.

2. Verfahren nach Anspruch 1, wobei jede der elastischen Strukturen **(1a, 1a', 1b, 1b')** ein erstes Bahnmaterial (**4a**) und ein zweites Bahnmaterial (**4b**) mit dazwischenliegenden elastischen Elementen (**16**) umfasst.

3. Verfahren nach einem der vorhergehenden Ansprüche, wobei es sich bei den beiden elastischen Strukturen **(1a, 1b)** um Längsendabschnitte eines Einfachbandes (**1, 100**) handelt, wobei die elastischen Strukturen (**1a, 1b**) einander durch Falten (**30**) des Einfachbandes (**100**) entlang seiner Längsachse (**X10**) überlappen.

4. Verfahren nach Anspruch 1 oder 2, wobei die beiden elastischen Strukturen **(1a', 1b')** in Querrichtung durch diskrete Elemente (2) verbunden werden, die gleiche Abstände **(P1')** zueinander aufweisen und um eine Querachse (**X2**) gefaltet werden, wobei das Verfahren den Schritt des Bildens einer Verbundbandstruktur (**100'**) umfasst, wobei die elastischen Strukturen (**1a', 1b'**) aufeinander gelegt werden, wobei Abschnitte der diskreten Elemente (**2**) zwischen den gefalteten elastischen Strukturen (**1a', 1b'**) angeordnet werden.

5. Verfahren nach einem der vorhergehenden Ansprüche, umfassend einen technischen Schweißschritt (**180**) der überlappenden (**30, 610**) elastischen Strukturen **(1a, 1a', 1b, 1b')** vor dem Verlangsamungsschritt (**70**).

6. Vorrichtung zum Ausführen des Verfahrens nach einem der vorhergehenden Ansprüche, wobei die Vorrichtung Folgendes umfasst:
- eine Bremseinrichtung (**70**), die geeignet ist, das Verbundband (**100,** 100') von einer ersten Geschwindigkeit (**V1**) auf eine zweite Geschwindigkeit (**V2**) zu verlangsamen, und
- eine Schweißeinrichtung (**80**), die geeignet ist, auf dem sich mit der zweiten Geschwindigkeit (**V2**) bewegenden Verbundband (**100, 100'**) quer zu der Längszuführrichtung (**MD**) verlaufende Schweißlinien (**13**) zu erzeugen,
**dadurch gekennzeichnet, dass** die Bremseinrichtung (**70**) einen sich mit der ersten Geschwindigkeit (**V1**) bewegenden ersten motorisierten Bandförderer (**73**) und einen sich mit der zweiten Geschwindigkeit (**V2**) bewegenden zweiten motorisierten Bandförderer (**78**) umfasst, wobei der erste und der zweite Bandförderer (**73, 78**) in Längsrichtung beabstandet sind.

7. Vorrichtung nach Anspruch 6, umfassend eine Schweißeinrichtung (**180**), die geeignet ist, auf der ersten und der zweiten überlappenden Struktur (**1a**, **1a', 1b, 1b')** auf dem sich mit der ersten Geschwindigkeit (**V1**) bewegenden Verbundband (**100, 100'**) technische Schweißungen zu bilden, wobei die Schweißeinrichtung (**180**) der Bremseinrichtung (**70**) vorgelagert ist.

## Revendications

1. Procédé de soudage transversal d'un ruban composite (100, 100') comprenant les étapes consistant à :
- former un ruban composite (100, 100') comprenant des première et deuxième structures élastiques (1a, 1a', 1b, 1b') se chevauchant, qui est avancé en continu dans une direction longitudinale (MD) à une première vitesse d'avance (V1) et à une première tension d'avance (T1) ;
- ralentir (70) ledit ruban composite (100), l'amenant à une deuxième vitesse (V2) et à une deuxième tension (T2), respectivement inférieures à ladite première vitesse d'avance (V1) et à ladite première tension d'avance (T1),
- avancer ledit ruban composite (100, 100') auxdites deuxièmes vitesse (V2) et tension (T2) jusqu'à un poste de soudage (80) et souder (80) ledit ruban composite (100, 100') avec des lignes de soudure (13) transversales à ladite direction d'avance longitudinale (MD), et
- avancer ledit ruban composite (100, 100') auxdites deuxièmes vitesse (V2) et tension (T2) jusqu'à un poste de coupe (90) et couper (90) ledit ruban composite (100, 100') avec des coupes (12) transversales à ladite direction d'avance longitudinale (MD),
**caractérisé en ce que** ladite étape de ralentissement dudit ruban composite (100, 100') est effectuée en avançant ledit ruban composite (100, 100') vers un premier convoyeur à bande motorisé (73) se déplaçant à ladite première vitesse (V1) et ensuite à un deuxième convoyeur à bande motorisé (78) se déplaçant à ladite deuxième vitesse (V2), dans lequel lesdits premier et deuxième convoyeurs à bande (73, 78) sont espacés dans la direction longitudinale.

2. Procédé selon la revendication 1, dans lequel chacune desdites structures élastiques (1a, 1a', 1b, 1b') comprend un premier matériau en bande (4a) et un deuxième matériau en bande (4b) avec des éléments élastiques interposés (16).

3. Procédé selon l'une quelconque des revendications précédentes, dans lequel les deux structures élastiques (1a, 1b) sont des parties d'extrémité longitudinale d'un même ruban (1, 100), où lesdites structures élastiques (1a, 1b) se chevauchent par pliage (30) dudit ruban unique (100) le long de son axe longitudinal (X10).

4. Procédé selon les revendications 1 ou 2, dans lequel les deux structures élastiques (1a', 1b') sont assemblées transversalement par des éléments discrets (2) équidistants (P1') l'un de l'autre et pliés autour d'un axe transversal (X2), où le procédé comprend l'étape de formation d'une structure de ruban composite (100'), où lesdites structures élastiques (1a', 1b') sont appliquées l'une au-dessus de l'autre avec des parties desdits éléments discrets (2) agencées entre lesdites structures élastiques pliées (1a', 1b').

5. Procédé selon l'une quelconque des revendications précédentes, comprenant une étape de soudage technique (180) desdites structures élastiques (1a, 1a', 1b, 1b') chevauchantes (30, 610) avant ladite étape de ralentissement (70).

6. Appareil pour mettre en œuvre le procédé selon l'une quelconque des revendications précédentes, ledit appareil comprenant :
- un dispositif de freinage (70) adapté pour ralentir ledit ruban composite (100, 100') d'une première vitesse (V1) à une deuxième vitesse (V2), et
- un dispositif de soudage (80) adapté pour réaliser des lignes de soudure (13) transversales à ladite direction d'avance longitudinale (MD) sur ledit ruban composite (100, 100') se déplaçant à ladite deuxième vitesse (V2),
**caractérisé en ce que** ledit dispositif de freinage (70) comprend un premier convoyeur à bande motorisé (73) se déplaçant à ladite première vitesse (V1) et un deuxième convoyeur à bande motorisé (78) se déplaçant à ladite deuxième vitesse (V2), où lesdits premier et deuxième convoyeurs à bande (73, 78) sont espacés dans la direction longitudinale.

7. Appareil selon la revendication 6, comprenant un dispositif de soudage (180) adapté pour former des soudures techniques sur lesdites première et deuxième structures élastiques (1a, 1a', 1b, 1b') chevauchantes sur ledit ruban composite (100, 100') se déplaçant à ladite première vitesse (V1), ledit dispositif de soudage (180) étant situé en amont dudit dispositif de freinage (70).
